Europäisches Patentamt

European Patent Office

Office européen des brevets

(19)

(11) Veröffentlichungsnummer: **0 303 195 B1**

(12) # EUROPÄISCHE PATENTSCHRIFT

(45) Veröffentlichungstag der Patentschrift: 30.10.91

(51) Int. Cl.⁵: **A61F 2/38**

(21) Anmeldenummer: **88112766.6**

(22) Anmeldetag: **05.08.88**

(54) Endoprothese für ein Kniegelenk.

(30) Priorität: **13.08.87 DE 3726969**

(43) Veröffentlichungstag der Anmeldung:
**15.02.89 Patentblatt 89/07**

(45) Bekanntmachung des Hinweises auf die
Patenterteilung:
**30.10.91 Patentblatt 91/44**

(84) Benannte Vertragsstaaten:
**AT BE CH DE ES FR GB GR IT LI LU NL SE**

(56) Entgegenhaltungen:
**EP-A- 0 069 683**     **EP-A- 0 183 670**
**DE-A- 2 549 318**     **DE-A- 2 901 009**
**FR-A- 2 094 755**     **FR-A- 2 330 377**
**FR-A- 2 502 937**     **FR-A- 2 566 657**
**GB-A- 1 567 007**     **US-A- 3 932 045**
**US-A- 4 216 549**     **US-A- 4 586 933**

(73) Patentinhaber: **Friedrichsfeld Aktiengesellschaft Keramik- und Kunststoffwerke
Steinzeugstrasse 50
W-6800 Mannheim 71(DE)**

(72) Erfinder: **Rehder, Günther
Neekoppel 7
W-2300 Kiel 14(DE)**

(74) Vertreter: **Klose, Hans, Dipl.-Phys. et al
Kurfürstenstrasse 32
W-6700 Ludwigshafen(DE)**

**Beschreibung**

Die Erfindung betrifft eine Endoprothese für ein Kniegelenk mit einem Femurteil, einem Tibiateil und einem Mittelteil, welcher mit dem Femurteil über einen bevorzugt horizontal angeordneten Bolzen bewegbar verbunden ist, wobei der Femurteil gegenüber dem Tibiateil um eine im wesentlichen horizontale Beugeachse schwenkbar ist und ein gekrümmtes Lagerelement aufweist, sowie mit einem zwischen dem Mittelteil und dem Tibiateil angeordneten Zentrierzapfen, um welchen der Mittelteil gegenüber dem Tibiateil drehbar gelagert ist, und ferner mit einem vom Beugewinkel abhängigen Anschlag für den Femurteil, dessen Drehbewegung bezüglich des Zentrierzapfens in der Strecklage begrenzt ist.

Aus der DE-A-25 49 318 ist eine derartige Endoprothese mit einem Femurteil, einem Zwischenteil und einem Tibiateil nachgewiesen, das einen Bewegungsbereich um die Beugeachse von -5° bis +145° aufweist. Die Unterschenkelrotation ist mittels des Beugeachsbolzens steuerbar, dessen Mittelbereich einem beugewinkelabhängigen Anschlag für die Drehbewegung des mit dem Tibiateil drehfest verbundenen Zentrierzapfens bildet. Hierzu ist der Mittelteil als ein exzentrischer Rotationskörper ausgebildet, welcher an einer Aussparung des Zentrierzapfens anliegt, wobei die Zapfenachse zur Beugeachse beabstandet ist und zu dieser im wesentlichen unter einem rechten Winkel angeordnet ist. Der Mittelteil weist zur Beugeachse koaxiale Lagerflächen für ebenfalls koaxiale Lagerschalen des Femurteiles auf. Die definierte Vorgabe der Beugeachse unabhängig vom Beugewinkel entspricht nicht der physiologischen Kinematik. Es besteht die Gefahr, daß infolge nur linienförmiger und in Extremfällen gar punktförmiger Anlage der Lagerflächen zwischen Femurteil und Tibiateil sehr hohe unzulässige Spitzenbelastungen auftreten, die nachteilig hinsichtlich Lebensdauer und Funktionsfähigkeit sind.

In dem Buch "Biomechanics of the Knee, Paul G. J. Maquet, Springer-Verlag, Berlin Heidelberg New York 1976" sind der Aufbau und die Funktion des Kniegelenkes im einzelnen dargelegt. Insbesondere auf den Seiten 32 bis 39 dieses Buches ist an Hand von Fig. 35 die Verlagerung der Beugeachse in Abhängigkeit des Beugewinkels im einzelnen erläutert. Eine einfache Scharnierbewegung zwischen Femurteil und Tibiateil wird dieser besonderen physiologischen Kinematik und Statik nicht gerecht, wodurch sich in der Vergangenheit nicht unerhebliche Schwierigkeiten hinsichtlich Funktionssicherheit, Lebensdauer, Belastbarkeit usw. ergaben.

Aus der DE-A-25 50 704 ist eine Kniegelenk-Endoprothese bekannt, in welcher zwei künstliche Meniskuselemente zwischen den gekrümmten Lagerflächen des Femurteils und den im wesentlichen ebenen Lagerflächen des Tibiateils angeordnet sind. Eine Zwangsführung der künstlichen Meniskuselemente ist nicht vorhanden, sondern es soll eine Selbstausrichtung mittels den Bändern und Muskeln erfolgen. Die gekrümmten Lagerflächen und ebenso der Meniskuselemente erfordern einen hohen Fertigungsaufwand, wobei infolge von Fertigungstoleranzen hohe Flächenpressungen in der Praxis schwer zu vermeiden sind. Es wird im übrigen ein voll funktionsfähiger Bewegungsapparat vorausgesetzt, so daß bei einer erheblichen Beschädigung desselben die Endoprothese gar nicht zum Einsatz gelangen kann. Um eine gegenseitige Rotation von Femur und Tibia beim Strecken und Beugen zu ermöglichen, müssen die gebogenen Lagerflächen des Femurteils und der beiden Meniskuselemente sphärisch gekrümmt ausgebildet sein, wodurch ein erheblicher Fertigungsaufwand bedingt ist. Schon geringfügige Abweichungen in der Geometrie der Lagerflächen können zu hohen Flächenpressungen und Spitzenbelastungen führen, mit der Folge von einer schnellen Abnutzung und Beschädigungen. Die Verwendung von besonderen, nachgiebigen Werkstoffen für das Meniskuselement erfordert zusätzliche Maßnahmen und gleichwohl einen hohen Fertigungsaufwand der Lagerflächen.

Ferner ist aus der DE-A-24 52 412 eine Endoprothese nachgewiesen, deren Femurteil kufenartige, gebogene Abrollflächen aufweist. Der Krümmungsradius dieser Abrollflächen nimmt in dorsaler Richtung ab. Auf dem Tibiateil sind zwei Zwischenelemente fest und unverschiebbar angeordnet, welche sphärisch gekrümmte Lagerflächen für die Abrollflächen des Femurteiles aufweisen. Nur über einen kleinen Anteil der Gesamtfläche liegen die Abrollflächen des Femurteils an den Lagerflächen des Tibiateils an, so daß auch hier mit großen Flächenpressungen gerecht werden muß. Die Drehbarkeit zwischen Femurteil und Tibiateil wird durch die sphärische Ausbildung der genannten Flächen ermöglicht, wodurch jedoch ein nicht unerheblicher Fertigungsaufwand erforderlich ist.

Der Erfindung liegt daher die Aufgabe zugrunde, die Endoprothese der eingangs genannten Art dahingehend weiterzubilden, daß bei geringem Fertigungsaufwand die Kräfte auf möglichst großen Flächen übertragbar sind und die physiologischen Kniegelenkfunktionen bei Bewegung und Belastung erfüllt werden.

Die Lösung dieser Aufgabe erfolgt gemäß den im Kennzeichen des Patentanspruchs 1 angegebenen Merkmalen.

Die erfindungsgemäße Endoprothese gewährleistet bei zuverlässiger und kompakter Konstruktion eine optimale Realisierung der physiologischen Kinematik und Statik, wobei örtliche Belastungsspitzen, insbesondere infolge von Linien- oder Punktberührung, zuverlässig vermieden werden. Mittels des vorgeschlagenen Lagerelements werden die Bewegungsabläufe zum einen in die rotatorische Bewegung mittels des ersten Führungskörpers und in eine rein translatorische Bewegung mittels des zweiten Führungskörpers unterteilt. Die Bewegung der Beugeachse erfolgt entlang einer evolutenartigen Kurve aufgrund der Führung des Bolzens in der Ausnehmung, wobei während des Beugens die Verschiebung des Mittelteils erzwungen wird und eine ständige Anlage der beiden Führungskörper an der Lagerbahn bzw. Führungsbahn sichergestellt wird. Der Bolzen ist exzentrisch zur Mittenachse des kreisförmigen Lagerelements angeordnet. Aufgrund des Achsversatzes wird in Verbindung mit der Führung des Bolzens der Lagerteil zwangsweise nachgeführt. Die Endoprothese weist kleine Abmessungen auf und die physiologische Kinematik und Statik kann unabhängig von der Funktionsfähigkeit des Band- und Muskelapparates sichergestellt werden. Es werden gute tribologische Eigenschaften hinsichtlich der gegeneinander bewegbaren Teile sowie eine hohe statische Festigkeit der Kräfte übertragenden Teile gewährleistet. Darüber hinaus werden Reibungskräfte zwischen den einzelnen gegeneinander bewegbaren Bauteilen minimiert, und zwar insbesondere im Hinblick auf die Haftreibung bei Beginn einer Bewegung. Die Bewegung der Beugeachse erfolgt entlang einer evolutenartigen Kurve aufgrund der Führung des Bolzens in einer Ausnehmung, insbesondere in Form eines Langloches oder dergleichen, wobei während des Beugens die Verschiebung des Lagerteiles erzwungen wird und eine ständige Anlage der beiden Führungskörper an dem Lagerelement sichergestellt wird. Erfindungsgemäß ist der Bolzen exzentrisch zum kreisförmigen Lagerelement angeordnet, das in zweckmäßiger Weise als eine zylindrische Lagerbahn ausgebildet ist. Aufgrund des erfindungsgemäßen Achsversatzes wird in Verbindung mit der Führung des Bolzens der Lagerteil beim Beugen zwangsweise nachgeführt. Die genannte Führung wird vor allem als eine evolutenartige Kurve ausgebildet, doch kann im Rahmen der Erfindung die Führung auch als ein gerades Langloch, Nut oder dergleichen ausgebildet sein, um den erfindungsgemäßen Achsausgleich beim Beugen zu ermöglichen.

Obgleich der Bolzen zweckmäßig auf dem Femurteil und die Führung auf dem Mittel-bzw. Tibiateil angeordnet sind, kann im Rahmen der Erfindung auch die umgekehrte Anordnung in entsprechender Weise vorgesehen sein. Die Lagerbahn ist erfindungsgemäß kreisförmig, insbesondere nach Art eines Zylinders ausgebildet und der genannte Bolzen ist exzentrisch zur Lagerbahn angeordnet. Aufgrund der erfindungsgemäß exzentrischen Anlenkung des Mittelteiles und dessen Führung wird beim Beugen die Verschiebung des Lagerteiles mittels der Führung zum Achsausgleich erzwungen. Hierbei findet eine Roll- Gleitbewegung statt, die zweckmäßig auf einen vorgebbaren Beugewinkelbereich mittels der Führung eingeschränkt werden kann, wobei nachfolgend ausschließlich eine Rollbewegung stattfindet und die Translationsbewegung unterbleibt und gegebenenfalls aus wieder in der entgegengesetzten Richtung erfolgen kann. Insbesondere durch die Form und die Größe der Ausnehmung, welche zweckmäßig als ein Langloch ausgebildet ist, kann die Grenze des Winkelbereiches für Rotation-Translationsbewegung auf den genannten Winkel vorgegeben werden, der insbesondere zwischen 85 und 98 Grad liegt. Die Führungskörper werden im Hinblick auf Minimierung der Reibung zweckmäßigerweise als Wälzlager ausgebildet. Das Lagerelement bzw. die Lagerbahn des Femurteiles und/oder die Führungsbahn des Mittelteiles sind zweckmäßigerweise als hinterschnittene Nuten ausgebildet, in welche die Führungskörper entsprechend eingreifen, so daß Zug- und Druckbelastungen funktionssicher aufgenommen werden. Im Rahmen der Erfindung können die Führungskörper auf dem Femurteil bzw. dem Mittelteil angeordnet sein, während der Lagerteil entsprechend die koaxiale Lagerbahn und/oder die Führungsbahn aufweist.

In einer besonderen Ausgestaltung enthält der Lagerteil wenigstens einen in eine Steuerbahn des Tibiateiles eingreifenden Bolzen oder umgekehrt zur Bildung des beugewinkelabhängigen Anschlages. Ist beispielsweise der Bolzen auf dem Lagerteil angeordnet, so wird bei dessen Translationsbewegung ebenfalls der Bolzen bewegt. Die Steuerbahn ist in der Weise ausgebildet, daß in der Strecklage eine Drehbewegung des Tibiateiles blockiert wird und mit zunehmendem Beugungswinkel auch der Drehwinkel des Tibiateiles zunimmt.

Gemäß einer besonderen Ausgestaltung enthält der Femurteil einen intrakondylären Käfig sowie zwei seitlich angeordneten Kondylenrollen, mit den Führungselementen oder Lagerbahnen für den ersten Führungskörper. Der Käfig weist einen etwa U-förmigen, zum Tibiateil sich öffnenden Querschnitt auf. Durch die in der Mitte sich befindliche Öffnung kann eine erfindungsgemäße Zugstange hindurchgeführt werden zur Verankerung im Femur. Ferner wird der Käfig mittels Kompressionsplatten im Kondylus befestigt, wobei die Verschraubung von der genannten mittleren Öffnung her vorgenommen wird. Auch der Tibiateil enthält zweckmäßigerweise einen Käfig, der mittels einer Zugstange und einen in die Corticalis geschraubten Schraubkörper befestigt wird. Die Zugstange für den Tibiakäfig und ebenso auch die für den intrakondylaren Käfig liegen mit kugelförmigen Flächen an ebenfalls kugelförmigen Flächen in den Öffnungen der genannten Käfige an, wodurch problemlos ein Achsenausgleich ermöglicht wird. Der Schraubkörper weist auf seiner Außenfläche zweckmäßig ein selbstschneidendes Gewinde auf, das vorzugsweise als ein Sägezahngewinde ausgebildet ist. Die Zugstange ist in ein Innengewinde mit einem Anschlag derart eingeschraubt, daß nach dem vollständigen Einschrauben beim Drehen der Zugstange auch der Schraubkörper gedreht und damit in die Corticalis einschraubbar ist. Im Rahmen der Erfindung ist in entsprechender Weise auch der Tibiateil verankert. Diese Art der Befestigung von Gelenkteilen kann außer der bisher erläuterten Endoprothese auch bei anderen Gelenken, insbesondere im Hüftgelenk, im Rahmen dieser Erfindung zum Einsatz gelangen. Alternativ können Femurteil und Tibiateil auch in gewohnter Weise einzementiert sein.

In einer wesentlichen Ausgestaltung ist der tibiale Käfig über wenigstens eine Verankerungskralle in der Corticalis abgestützt. Diese Verankerungskralle ist in einer bevorzugt schwalbenschwanzförmigen Führungsbahn des Käfigs verschiebbar, wobei mittels einer Druckschraube der Abstand zum Käfig und folglich ein guter Drucksitz an der Corticalis gewährleistet wird.

Die erläuterte Verankerung der Käfige mittels Kompressionsplatten bzw. Verankerungskrallen sowie Zugstangen kann im Rahmen dieser Erfindung nicht nur bei einem Kniegelenk sondern entsprechend auch bei einem Hüftgelenk vorgesehen werden. In beiden Anwendungsfällen wird eine zuverlässige Verankerung der Gelenkteile sichergestellt. Mittels den erläuterten Verankerungskrallen und den Schrauben kann der Käfig in optimaler Weise bezüglich des Knochens justiert werden. Ferner kann durch unterschiedliche Größen und Formen der genannten Verankerungskrallen in einfacher Weise eine Anpassung an die jeweiligen Verhältnisse selbst intraoperativ vorgenommen werden, wobei gleichwohl eine funktionssichere Verankerung gewährleistet wird.

Weitere wesentliche Ausgestaltungen und Merkmale sind in der nachfolgenden Beschreibung von Ausführungsbeispielen sowie in den Patentansprüchen angegeben.

Die Erfindung wird anhand von in der Zeichnung dargestellten Ausführungsbeispielen näher erläutert. Es zeigen:

Fig. 1    einen Längsschnitt der Kniegelenk-Endoprothese in der Streckstellung und in der maximalen Beugestellung,

Fig. 2    eine explosionsartige Darstellung des Femurteiles, und zwar des intra

kondylaren Käfigs und einer Kondylenrolle sowie des Lagerteiles,

Fig. 3    eine seitliche Ansicht des intrakondylären femuralen Käfigs und der beiden Kondylenrollen in Blickrichtung III gemäß Fig. 2,

Fig. 4    eine explosionsartige Darstellung des Mittelteiles sowie des tibialen Käfigs,

Fig. 5    eine Ansicht in Blickrichtung VI gemäß Fig. 4,

Fig. 6    eine Ansicht des tibialen Käfigs in Blickrichtung VI gemäß Fig. 4,

Fig. 7    eine Aufsicht auf den tibialen Käfig in Blickrichtung VII gemäß Fig. 4, wobei auch die Bolzen des Lagerteils angedeutet sind,

Fig.8a-f    verschiedene Ausführungsformen der Führungen des Lagerteils bezüglich der Kondylenrolle sowie des Mittelteils beim Einsatz unterschiedlicher Werkstoffe.

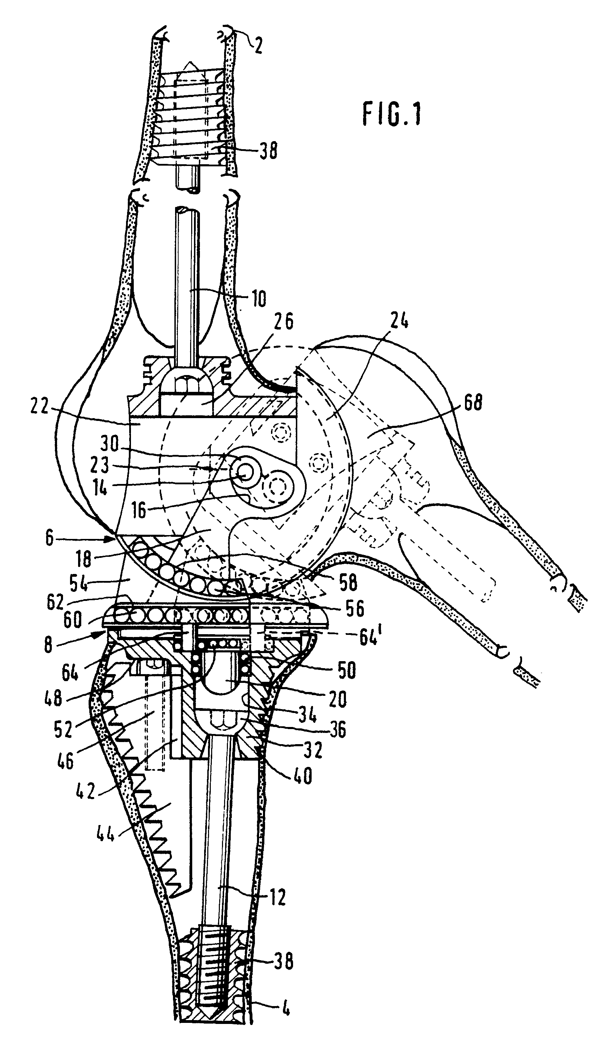

In Fig. 1 sind Femur 2 und Tibia 4 teilweise dargestellt, in welchen der Femurteil 6 bzw. der Tibiateil 8 mittels Zugstangen 10, 12 verankert sind. Der Femurteil 6 enthält einen Bolzen 14, welcher in einer als Langloch ausgebildeten Ausnehmung 16 eines Mittelteiles 18 geführt ist. Der Mittelteil 18 weist einen in den Tibiateil 8 eingreifenden Zentrierzapfen 20 auf, wobei die Rotation des Tibiateiles 8 und somit des Unterschenkels um diesen Zentrierzapfen 20 in Abhängigkeit von der Beugung gesteuert wird. Durch die dargestellte Ausbildung der Führung des Bolzens 14 in der Ausnehmung 16, die als Langloch ausgebildet ist, erfolgt insoweit keine Einschränkung. Zur Führung kann im Rahmen der Erfindung ein geradliniges Langloch, eine gerade Nut oder auch ein mit einer anderen Krümmung versehenes Langloch vorgesehen sein.

Der Femurteil 6 enthält einen intrakondylären Käfig 22 sowie zwei zu beiden Seiten angeordnete Kondylenrollen 24, wobei hier nur die eine hinter der Zeichenebene liegende Kondylenrolle zu erkennen ist. Die Kondylenrollen sind mittels Schrauben mit dem Käfig 22 verbunden. Die Kondylenrollen 24 weisen erfindungsgemäß kreisförmig gekrümmte Lagerelemente auf, die nachfolgend im einzelnen noch erläutert werden. Diese sind zweckmäßig als Lagerbahnen, bevorzugt als Teile von Zylinderflächen, ausgebildet, die koaxial zu einer gemeinsamen Mittenachse 23 liegen. Diese Mittenachse 23 bildet die Beugeachse der Endoprothese, wobei erfindungsgemäß der Bolzen 14 in einem vorgegebenen Abstand exzentrisch zur Mittenachse 23 angeordnet ist. Beim Beugen erfolgt somit zwangsweise eine Bewegung und Verschiebung des Mittelteiles, der über den Bolzen exzentrisch zur Beugeachse am Femurteil angelenkt ist. Das nachfolgend noch zu erläuternde Lagerteil, welches als künstlicher Meniskus wirksam ist, erzwingt eine weitere Bewegung. Betrachtet man den Tibiateil als feststehend, so wird aufgrund der Zwangsführung des Mittelteiles über den exzentrischen Bolzen im Femurteil eine Zwangsführung der Beugeachse entlang einer evolutenartigen Kurve erreicht. Durch die Formgebung der Außenfläche des Bolzens und/oder der Geometrie der Ausnehmung 16 wird erfindungsgemäß die Bewegung der Beugeachse entlang einer evolutenartigen Kurve entsprechend dem physiologischen Bewegungsablauf erzwungen. Hierzu sind keine sphärischen Lagerbahnen, über welche die von der Endoprothese aufzunehmenden Kräfte übertragen werden, erforderlich. Die als Langloch ausgebildete Ausnehmung 16 und der eingreifende Bolzen dienen nicht zur Kraftübertragung, sondern zur zwangsweisen Steuerung und Bewegung des Mittelteiles 18 und des Lagerteiles 54. Der Käfig 22 ist etwa U-förmig ausgebildet und enthält in der Mitte eine Öffnung 26, die sich nach unten zum Tibiateil öffnet und in welche der Mittelteil 18 mit einem Arm 28 erfindungsgemäß hineinragt. Am oberen Ende dieses Armes 28 ist das Langloch 16 angeordnet, wobei mittels eines hier schematisch angedeuteten Kugellagers 30 der im Käfig 22 befestigte Bolzen 14 geführt ist. Dieses Langloch 16 ist erfindungsgemäß in der Weise gekrümmt, daß beim Beugen die Beugeachse bezüglich des Tibiateiles die bereits erläuterte Evolute durchführt. Mittels gestrichelten Linien ist die maximale Beugestellung angedeutet.

Der Tibiateil 8 enthält einen tibialen Käfig 32, der in der Mitte eine Bohrung 34 mit kugelförmiger Bodenfläche aufweist. Der Schraubenkopf der tibialen Zugstange 12, entsprechendes gilt auch für die femurale Zugstange 10 sowie den intrakondylären Käfig 22, weist eine entsprechend kugelförmig ausgebildete Anlagefläche auf, so daß ein Achsausgleich vorgenommen werden kann. Das andere Ende der Zugstange 12 ist in einen Schraubkörper 38 geschraubt, der an der Außenfläche ein selbstschneidendes Gewinde zum Verankern in der Tibia aufweist. Der Käfig 32 weist auf einer Seite Krallen 40 auf, die fest in die Innenfläche der Tibia 4 eingreifen. Auf der gegenüberliegenden Seite enthält der Käfig 32 eine als Schwalbenschwanz ausgeführte Führungsnut 42 für eine Verankerungskralle 44, in welche eine Druckschraube 46, insbesondere mittels Linksgewinde, eingeschraubt ist. Der Kopf dieser Druckschraube 46 liegt an einer Schulter 48 des Tibiateiles unten an und aufgrund des genannten Linksgewindes wird bei Drehen der Druckschraube 46 im Uhrzeigersinn der Abstand zwischen der Schulter 48 und der Verankerungskralle 44 vergrößert. Wie nachfolgend noch zu erläutern sein wird, sind entsprechend vor und hinter der Zeichenebene zwei diametral einandergegenüberliegende weitere Verankerungskrallen vorgesehen. Mittels diesen Verankerungskrallen kann erfindungsgemäß eine zuverlässige Befestigung des Käfigs 32 erfolgen, wobei mittels den Druckschrauben die erforderliche Ausrichtung und Anpressung erreicht wird.

Der Zentrierzapfen 20 ist mit dem Mittelteil 18 fest verbunden und ragt in die Bohrung 34 des tibialen Käfigs 32. Zur radialen bzw. axialen Lagerung sind die Kugellager 50, 52 zwecks reibungsarmer Lagerung vorgesehen. Auf dem Mittelteil 18 ist ferner zu beiden Seiten des Armes 28 jeweils ein Lagerteil 54 verschiebbar angeordnet. Diese Lagerteile 54 sind als künstliche Menisken ausgebildet und in einer Ebene, die parallel zur Beugeachse und ferner orthogonal zur Achse des Zentrierzapfens 20 steht, verschiebbar. Jedes Lagerteil 54 weist einen ersten Führungskörper 56 auf, der hier als Kugellager ausgebildet ist und in das als Lagerbahn ausgebildete Lagerelement 58 des Femurteiles bzw. der Kondylenrolle 24 eingreift. Der erfindungsgemäße Führungskörper 56 ist ebenso wie die zugeordnete Lagerbahn 58 koaxial zur Beugeachse angeordnet. An der Unterseite weist der Lagerteil 54 einen zweiten Führungskörper 60 auf, der ebenfalls als ein Wälzlager, also Kugel- bzw. Rollenlager, ausgebildet ist und in der Führungsbahn 62 des Mittelteiles eingreift. Durch die Ausbildung der Lagerbahn 58 sowie der Führungsbahn 62 jeweils als hinterschnittene Nuten wird die Führung der Kugeln oder Rollen erreicht. In der dargestellten Streckstellung befindet sich der künstliche Meniskus bzw. der Lagerteil 54 bezüglich der durch den Zentrierzapfen 20 vorgegebenen Rotationsachse auf der anderen Seite als der Bolzen 14. Wie durch die gestrichelten Linien angedeutet, wird der Lagerteil 54 beim Beugen infolge der Führung des Bolzens 14 in dem beschriebenen Langloch 16 gemäß Zeichnung nach rechts bewegt. Hierbei wird der an der Unterseite des Lagerteiles angeordnete Bolzen 64 in die durch 64' bezeichnete Stellung gebracht. Zwecks Erläuterung der Zusammenhänge ist in der Zeichnung der Bolzen 64 vollständig dargestellt, obgleich er tatsächlich hinter der durch die Mitte des Zentrierzapfens 20 laufenden Schnitt-und Zeichnungsebene angeordnet ist.

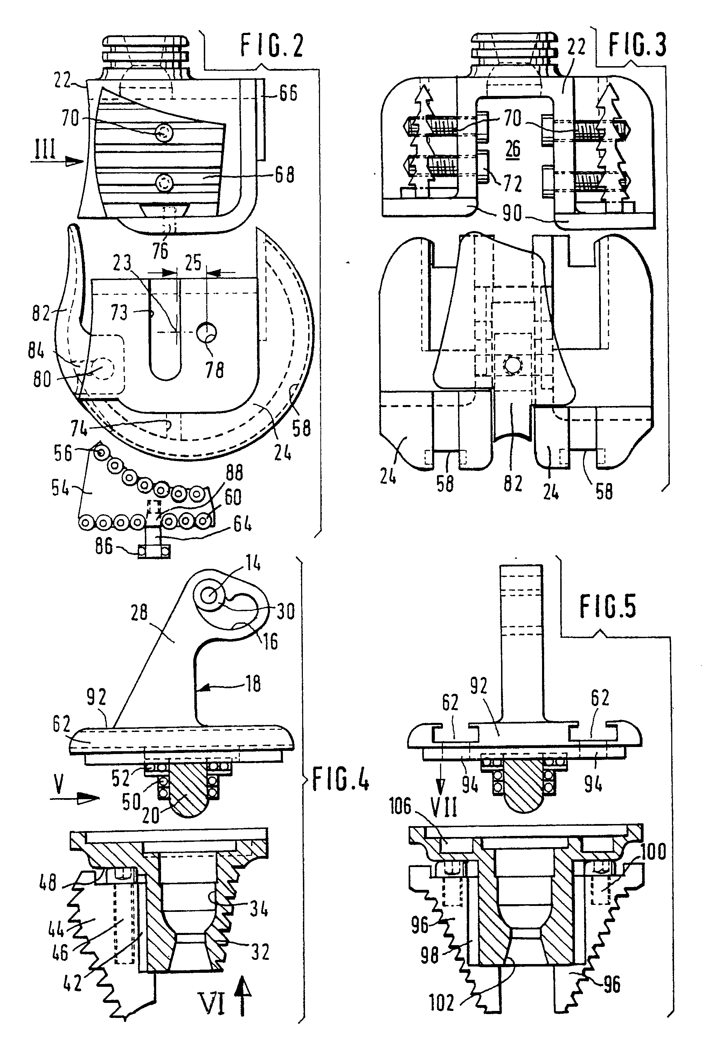

Fig. 2 zeigt in einer explosionsartigen Darstellung den intrakondylären Käfig 22, eine der beiden Kondylenrollen 24 sowie den Lagerteil 54, welcher einen künstlichen Meniskus bildet. Der Käfig 22 weist Kompressionsplatten auf einer Schwalbenschwanzführung 66 auf. Zur Befestigung der Kompressionsplatten 68 sind zwei Schrauben 70 in entsprechende Gewinde der Kompressionsplatten eingeschraubt, wobei die Schraubenköpfe in einer Nut 72 der Kondylenrolle zwecks Führung und Sicherung eingreifen. Die Befestigung der Kondylenrolle am Käfig 22 erfolgt über eine Schraube, welche durch eine Bohrung 74 der Kondylenrolle 24 hindurch in ein Gewinde 76 im Boden des Käfigs 22 eingreift.

Die Kondylenrolle 24 enthält ferner eine exzentrische Bohrung 78 zur Aufnahme des oben erläuterten Bolzens, welcher in die Ausnehmung bzw. das Langloch des Mittelteiles eingreift. Die Kondylenrolle 24 enthält eine Welle 80 zwecks justierbarer Lagerung des Patella-Gleitlagers 82, wobei nach der Positionierung mittels einer Schraube 84 die Fixierung erfolgt. Die Bohrung 78 weist zu den einzelnen Punkten der kreisförmigen Lagerbahn 58 unterschiedliche Abstände auf und duch die Anlenkung des Mittelteiles über den Bolzen wird beim Beugen die Nachführung zwecks Ausgleich des Achsversatzes erzwungen. Die Lagerbahn 58 und ebenso die Führungskörper 56 sind koaxial zur gemeinsamen Mittenachse 23, zu welcher die Bohrung 78 exzentrisch angeordnet ist. Es ist somit eine exzentrische Anlenkung des Mittelteiles bezüglich der Mittenachse 23 vorhanden, welche die Beugeachse definiert, so daß erfindungsgemäß beim Beugen eine gesteuerte Bewegung des Mittelteiles 18 in Abhängigkeit des Beugewinkels erfolgt. Entsprechend dem Abstand 25 der Bohrung 78 und somit des Bolzens von der Mittenachse 23 sowie der Formgebung, sei es der Außenfläche des Bolzens oder der genannten Ausnehmung, erfolgt ein evolutenartige Bewegung der Beugeachse.

In die Lagerbahn 58 greift der Lagerteil 54 mit seinem ersten Führungskörper 56, welcher erfindungsgemäß eine Anzahl von Wälzlagern aufweist, welche ebenso wie die Lagerbahn 58 koaxial zur Bohrung 78 angeordnet sind. Ferner sind die zweiten Führungskörper 60 als Wälzlager, also Kugel- oder Rollen-bzw. Nadellager ausgebildet. Auch der Bolzen 64 weist an seinem freien Ende ein Kugellager 86 auf, so daß insgesamt eine weitgehend reibungsfreie Führung ermöglicht wird. Der Bolzen 64 enthält erfindungsgemäß einen konischen Bund 88 zwecks Drehsicherung; ggfs. Können auch andere Mittel zur Sicherung des Bolzens 64 im Lagerteil 54 vorgesehen werden.

Fig. 3 zeigt den Käfig 22 bzw. die Kondylenrollen 24 in Blickrichtung III gemäß Fig. 2. Die in etwa U-förmige Ausgestaltung des Käfigs 22 mit der mittleren Öffnung 26, durch welche die Schrauben 70 geschraubt sind, ist gut zu erkennen. Auch die Schraubenköpfe 72, welche in die Nuten 73 der beiden Kondylenrollen 24 zur Führung und Sicherung eingreifen, sind dargestellt. Die Unterkanten des Käfigs 22 weisen einen Höhenversatz 90 entsprechend dem anatomischen Höhenversatz der Kondylen auf. Jede der beiden erfindungsgemäßen Kondylenrollen 24 enthält die Lagerbahn 58, welche als hinterschnittene Nut ausgebildet ist und die femurale Roll- oder Gleitbahn bildet.

Fig. 4 zeigt in gleicher Blickrichtung wie Fig. 2 den Mittelteil 18 und den tibialen Käfig 32. Am oberen Ende des Armes 28 ist die erfindungsgemäße Rollkurve in Form des Langloches 16 zu erkennen, in welcher der Bolzen 14 mittels des schematisch dargestellten Kugellagers 30 geführt ist. Im Plateau 92 des Mittelteiles 18 ist die Führungsbahn 62 durch gestrichelte Linien angedeutet. Auf dem Zapfen 20 sind zur radialen bzw. axialen Lagerung die beiden Kugellager 50, 52 angeordnet, welche auf entsprechend gegenüberliegenden Lagerflächen in der Bohrung 34 des tibialen Käfigs 32 laufen. Mittels der Druckschraube 46 kann der axiale Abstand zur Verankerungskralle 74 erfindungsgemäß vorgegeben werden. Mittels der als Langloch 16 ausgebildeten Führung wird beim Beugen die Nachführung des Mittelteiles 18 sowie des Lagerteiles 54 erzwungen. Hierbei erfolgt eine Rollbewegung und gleichzeitig auch eine Gleit-Translationsbewegung. Diese kombinierte Roll-Gleitbewegung kann im Rahmen der Erfindung auf einen maximalen Beugewinkel bis in die Größenordnung von 90 Grad, zweckmäßig zwischen 85 und 95 Grad begrenzt werden. Für größere Beugewinkel erfolgt dann ausschließlich eine Rotationsbewegung, wobei gegebenenfalls auch der Mittelteil 18 auch eine der ursprünglichen Bewegung entgegengesetzte Translationsbewegung ausführen wird. Der derart vorgegebene Grenzwinkel wird durch Formgebung, und zwar insbesondere die Länge des Langloches 16 oder allgemein der Führung des Bolzens 14 vorgegeben.

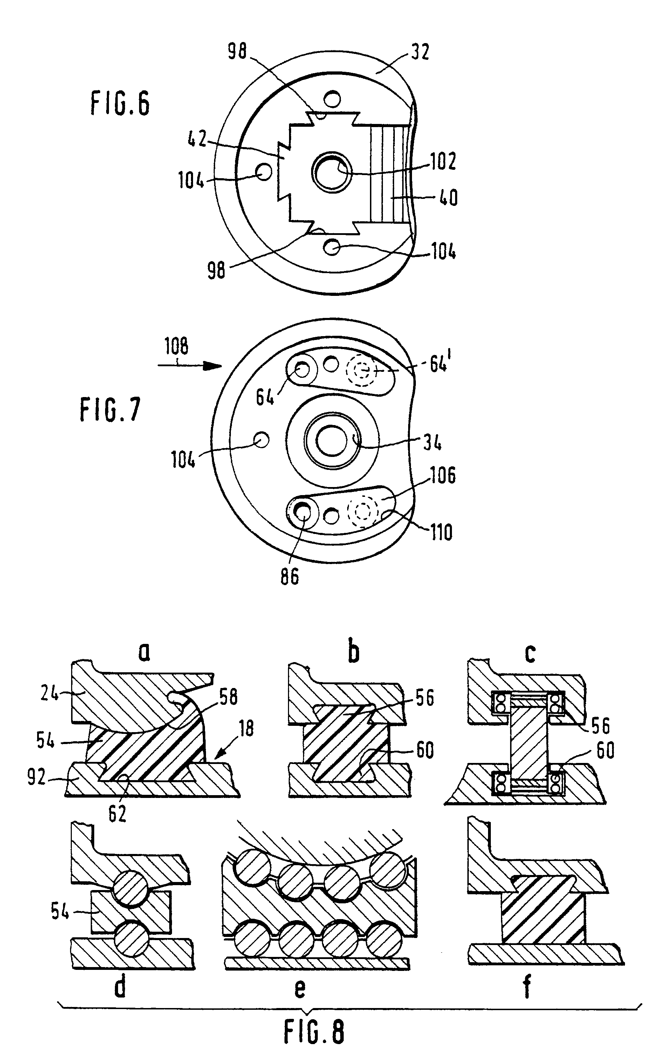

Fig. 5 zeigt in Blickrichtung V den Mittelteil 18 und den tibialen Käfig 32. Es sind nunmehr die beiden Führungsbahnen 62 für die beiden erfindungsgemäßen Lagerteile angeordnet. Den Führungsbahnen 62 ist erfindungsgemäß jeweils ein sich in der gleichen Richtung erstreckendes Langloch 94 zugeordnet, durch welches der in die Steuerbahn des tibialen Käfigs eingreifende Zapfen des Lagerteiles hindurchgreift. Es sind nunmehr auch die beiden anderen, einander diametral gegenüberliegenden Verankerungskrallen 96 zu erkennen, welche gleichfalls in Schwalbenschwanzführungen 98 des tibialen Käfigs 32 geführt sind. Die Druckschrauben 100 weisen ebenfalls ein Linksgewinde auf und dienen zur Veränderung des Abstandes und zur Verspannung der Verankerungskrallen 96 im Knochen.

Fig. 6 zeigt eine Ansicht des tibialen Käfigs in Blickrichtung VI gemäß Fig. 4, und zwar ohne die Verankerungskrallen. Die Führungsnuten 42 und 98, die hier schwalbenschwanzartig ausgebildet sind, sind ebenso wie die Krallen 40 zwecks unmittelbarer Anlage der Knocheninnenwand zu erkennen. Die Bohrung 102 ist im Rahmen der Erfindung konisch ausgebildet, um den Achsausgleich über die zugehörende Zugstange zu ermöglichen. Die Durchgangsbohrungen 104 sind für den Schraubenschlüssel zum Drehen der genannten Druckschrauben der Verankerungskrallen vorgesehen.

Fig. 7 zeigt eine Ansicht des tibialen Käfigs 32 in Blickrichtung VII gemäß Fig. 5, wobei nunmehr auch die beiden Steuerbahnen 106 für die Zapfen des jeweiligen Lagerteiles gut zu erkennen sind. Die Kugellager 86 sind zwecks Erläuterung der Zusammenhänge ebenfalls dargestellt. In der Streckstellung nehmen die beiden Kugellager 86 in der jeweiligen Steuerbahn 106 die gemäß Zeichnung Linke Endstellung und durch den derart gebildeten Anschlag wird eine Drehung des tibialen Käfigs 32 um die zur Zeichenebene senkrechte Achse blockiert. Erfindungsgemäß führen beim Beugen die beiden Lagerteile und folglich auch der Bolzen 64 mit den Lagern 86 jeweils eine Translationsbewegung senkrecht zum Zentrierzapfen aus, wie es durch den Pfeil 108 angedeutet ist. In der maximalen Beugestellung haben die beiden Zapfen die durch die gestrichelte Linie 64' angedeutete Position eingenommen und ermöglichen nunmehr die Rotation des Tibiateiles um einen Winkel von etwa 15°. Die beiden Steuerbahnen 106 sind von oben her in den Tibiateil 32 eingefräst, wobei die Außenkanten 110 koaxial zur Bohrung 34 liegen.

Fig. 8 zeigt unterschiedliche Ausgestaltungen von Kondylenrolle 24, Lagerteil 54, Mittelteil 18 sowie der entsprechenden Lager- bzw. Führungsbahnen 58 bzw. 62 einschließlich der Führungskörper 56, 60. Die verschiedenen Ausgestaltungen können in Abhängigkeit der zum Einsatz gelangenden Werkstoffe vorgegeben werden. Die Ausführungsformen gemäß Fig. 8a, 8b und 8f werden zweckmäßigerweise vorgesehen, wenn die Kondylenrolle und der Mittelteil 18 bzw. dessen Plateau 92 aus Chrom-Kobalt- Titan oder Keramik bestehen. Der Lagerteil 54 wird hierbei zumindest an seiner Oberfläche, zweckmäßigerweise insgesamt, aus einem Kunststoff, wie insbesondere Polyäthylen, bestehen. Die Ausführungsform gemäß Fig. 8c, d, e erweist sich bei Verwendung von Chrom-Kobalt sowie Titan als Kondylenrolle und Tibiaplateau zweckmäßig, wobei der Lagerteil 54 als Lagerbock für Kugel-oder Nadellager aus dem gleichen Material bestehen kann.

**Patentansprüche**

1. Endoprothese für ein Kniegelenk, mit einem Femurteil (6), einem Tibiateil (8) und einem Mittelteil (18), welcher mit dem Femurteil (6) über einen bevorzugt horizontal angeordneten Bolzen (14) bewegbar verbunden ist, wobei der Femurteil (6) gegenüber dem Tibiateil (8) um eine im wesentlichen horizontale Beugeachse schwenkbar ist und ein gekrümmtes Lagerelement (58) aufweist, und ferner mit einem zwischen dem Mittelteil (18) und dem Tibiateil (8) angeordneten Zentrierzapfen (20), um welchen der Mittelteil (18) gegenüber dem Tibiateil (8) drehbar gelagert ist, sowie mit einem vom Beugewinkel abhängigen Anschlag für den Femurteil (6), dessen Drehbewegung bezüglich des Zentrierzapfens (20) in der Strecklage begrenzt ist,
dadurch gekennzeichnet, daß zwischen dem Femurteil (6) und dem Mittelteil (18) ein als künstlicher Meniskus wirksamer Lagerteil (54) angeordnet ist,
daß der Lagerteil (54) einen ersten Führungskörper (56) aufweist, der mit dem kreisförmigen Lagerelement (58) des Femurteils (6) in Eingriff steht,
daß der Lagerteil (54) in einer zum Zentrierzapfen (20), insbesondere orthogonalen Ebene bezüglich des Mittelteiles (18) verschiebbar angeordnet ist und einen zweiten Führungskörper (60) aufweist, der mit einer Führungsbahn (62) des Mittelteils (18) in Eingriff steht,
daß der Bolzen (14) exzentrisch zur Mittenachse (23) des Lagerelements (58) des Femurteils angeordnet ist, wobei die Achse des Bolzens gegenüber der Mittenachse nach posterior verschoben ist,
und daß beim Beugen der Bolzen in einer Ausnehmung (16) des Mittelteils (18) entlang einer evolutenartigen Kurve bewegbar ist.

2. Endoprothese nach Anspruch 1, dadurch gekennzeichnet, daß das Lagerelement (58) des Femurteils (6) und die Führungskörper (56) des Lagerteils (54) koaxial zur Mittenachse (23) angeordnet sind, wobei der Bolzen (14) in einem Abstand (25) exzentrisch zur Mittenachse (23) angeordnet ist.

3. Endoprothese nach Anspruch 1 oder 2, dadurch gekennzeichnet, daß zur Bildung eines vom Beugewinkel abhängigen Anschlages der Lagerteil (54) wenigstens einen in eine Steuerbahn (106) des Tibiateils (8) eingreifenden Bolzen (64) oder umgekehrt aufweist.

4. Endoprothese nach einem der Ansprüche 1 bis 3, dadurch gekennzeichnet, daß die Führungsbahn (62) des Mittelteiles (18) im wesentlichen parallel zur Beugeachse angeordnet ist und/oder daß der Femurteil (6) einen intrakondylären Käfig (22) sowie zwei seitlich angeordnete Kondylenrollen (24) mit jeweils einem Lagerelement (58) aufweist.

5. Endoprothese nach einem der Ansprüche 1 bis 4, dadurch gekennzeichnet, daß der intrakondyläre Käfig (22) einen im wesentlichen U-förmigen Querschnitt mit einer zum Tibiateil (8) offenen Öffnung (26) aufweist und/oder daß der Käfig (22) mittels in Schlitzen des Kondylen eingebrachten Kompressionsplatten (68) verschraubt und befestigt ist.

6. Endoprothese nach einem der Ansprüche 1 bis 5, dadurch gekennzeichnet, daß die Führungskörper (56, 60) Wälzlager enthalten und/oder daß der Zentrierzapfen (20) mittels Wälzlager, die bevorzugt als axiale und radiale Kugellager (50, 52) ausgebildet sind, im Tibiateil (8) drehbar gelagert ist.

7. Endoprothese nach einem der Ansprüche 1 bis 6, dadurch gekennzeichnet, daß der erste Gelenk- oder Femurteil (6) und/oder der zweite Gelenk- oder Tibiateil (8) einen Käfig (22, 32) aufweisen, daß
der Käfig (22, 32) eine Bohrung für eine Zugstange (10, 12) aufweist, daß die Zugstange (10, 12) mit einer kugelförmigen Anlagefläche an einer entsprechend ausgebildeten Fläche des Käfigs (22, 32) anliegt und daß das freie Ende der Zustange (10, 12) in einen Schraubkörper (38) eingeschraubt ist, der an der Außenfläche ein selbstschneidendes Gewinde zur Verankerung in dem Knochen aufweist.

8. Endoprothese nach einem der Ansprüche 1 bis 7, dadurch gekennzeichnet, daß der Käfig (32) mittels Verankerungskrallen (44, 96) im Knochen abgestüzt ist und/oder zumindest an einem Teil seiner Außenfläche Krallen (40) aufweist, daß diese Verankerungskrallen (44, 96) in Führungen (66, 98), welche insbesondere als Schwalbenschwanzführungen ausgebildet sind, im Käfig (32) geführt sind, und daß mittels Druckschrauben (36, 100) der Abstand der Verankerungskrallen (44, 96) zum Käfig (32) vorgebbar ist.

9. Endoprothese nach einem der Ansprüche 1 bis 8, dadurch gekennzeichnet, daß der Mittelteil (18) auf der Unterseite eines Plateaus (92) den Zentrierzapfen (20) aufweist, der mittels Lagern (50, 52) in der Bohrung (34) des tibialen Käfigs (32) gelagert ist und/oder daß der Mittelteil (18) einen in die Öffnung (26) des intrakondylären Käfigs (22) eingreifenden Arm (28) aufweist, der an seinem oberen Ende die Rollkurve, die insbesondere als das Langloch (16) ausgebildet ist, aufweist.

10. Endoprothese nach einem der Ansprüche 1 bis 9, dadurch gekennzeichnet, daß die beiden Kondylenrollen (24) jeweils eine, zur Mittenachse (23) der Kondylenrolle (24) exzentrisch gelagerte Bohrung (78) zur Aufnahme des Bolzens (14) aufweist und/oder daß in der Öffnung (26) des intrakondylären Käfigs (22) auf dem Bolzen (14) ein Wälzlager (30) zur Führung im Langloch (16) angeordnet ist.

11. Endoprothese nach einem der Ansprüche 1 bis 10, dadurch gekennzeichnet, daß die beim Beugen stattfindende Roll-Gleitbewegung mittels der insbesondere als Langloch (16) ausgebildeten Führung des Bolzens (14) auf einen vorgebbaren Beugewinkel begrenzt wird, der zweckmäßig zwischen 85 und 95 Grad groß ist.

## Claims

1. An endoprosthesis for a knee joint, comprising a femur part (6), a tibia part (8), a middle part (18) movably connected to the femur part (6) via a pin (14) which is preferably horizontally mounted, the femur part (6) being pivotable relative to the tibia part (8) about a substantially horizontal flexion axis, and having a curved bearing element (58), and also a centering pin (20) arranged between the middle part (18) and the tibia part (8) about which the middle part (18) is pivotably mounted with respect to the tibia part (8), and a stop for the femur part (6) whose pivoting movement with respect to the centering pin (20) is limited in the extended position, said stop being dependent on the flexion angle, characterised in that a bearing part (54), which acts as an artificial meniscus, is arranged between the femur part (6) and the middle part (18), said bearing part (54) having a first guide element (56) engaging the circular bearing element (58) of the femur part (6) and being displaceable with respect to the middle part (18) in a plane orthogonal, in particular, to the centering pin (20) and having a second guide element (60) engaging a guide track (62) of the middle part (18), wherein said pin (14) is eccentrically mounted with respect to the centre axis (23) of the bearing member (58) of the femur part, the axis of the pin being set back relative to the centre axis, and that, upon flexing, the bolt is movable in a recess (16) in the middle part (18) along an evolute curve.

2. An endoprosthesis according to claim 1, characterised in that said bearing element (58) of the femur part (6) and said guide element (56) of the bearing part (54) are arranged coaxially with respect to the centre axis (23), and said pin (14) is arranged eccentrically to, and spaced a distance (25) from, said centre axis.

3. An endoprosthesis according to claim 1 or 2, characterised in that, in order to form a stop which is dependent on the flexion angle, the bearing part (54) has at least one pin (64) engaging in a control path (106) of the tibia part (8) or vice versa.

4. An endoprosthesis according to one of claims 1 to 3, characterised in that said guide track (62) of the middle part (18) is arranged essentially parallel to the flexion axis and/or in that said femur part (6) comprises an intracondylar cage (22) and two laterally arranged condyle rolls (24), each having a bearing element (58).

5. An endoprosthesis according to one of claims 1 to 4, characterised in that said intracondylar cage (22) has a substantially U-shaped cross-section with an opening (26) open towards the tibia part (8) and/or in that the cage (22) is screwed and fixed by means of compression plates (68) inserted into slots in the condyle.

6. An endoprosthesis according to one of claims 1 to 5, characterised in that the guide elements (56, 60) comprise roller bearings, and/or in that the centering pin (20) is pivotably mounted in the tibia part (8) by means of roller bearings, which are preferably in the form of axial and radial ball bearings (50, 52).

7. An endoprosthesis according to one of claims 1 to 6, characterised in that the first joint component or femur part (6) and/or the second joint component or tibia part (8) has a cage (22, 32), said cage (22, 32) has a bore for a connecting rod (10, 12) which bears, by means of a spherical bearing surface, on a corresponding surface of said cage (22, 32), and the free end of said connecting rod (10, 12) is screwed into a screw element (38) having a self-tapping external thread on its exterior surface for anchoring in a bone.

8. An endoprosthesis according to one of claims 1 to 7, characterised in that said cage (32) is secured in the bone by means of anchoring claws (44, 96), and/or has anchoring claws (40) over at least part of its outer surface, said anchoring claws (44, 96) being guided in said cage (32) in guides (66, 98), more particularly in the form of dovetail guides, and the spacing of the anchoring claws (44, 96) to the cage (32) is predeterminable by means of pressure screws (36, 100).

9. An endoprosthesis according to one of claims 1 to 8, characterised in that said middle part (18) carries said centering pin (20) on the underside of a plateau (92), and said centering pin (20) is mounted by means of bearings (50, 52) in a bore (34) of a tibial cage (32), and/or said middle part (18) has an arm (28) engaging in the opening (26) of the intracondylar cage (22), and said arm has, at an upper end thereof, a roll curve which is designed more particularly as an elongated slot (16).

10. An endoprosthesis according to one of claims 1 to 9, characterised in that said two condyle rolls (24) each have a bore (78) positioned eccentrically with respect to the centre axis (23) of the condyle roll (24) for receiving said pin (14), and/or a roller bearing (30) is arranged on said pin (14) in the opening (26) of the intracondylar cage (22) for guiding said pin in the elongated slot (16).

11. An endoprosthesis according to one of claims 1 to 10, characterised in that the rolling/sliding movement which takes place upon flexing of the joint is limited to a predeterminable flexion angle by means of the guide for said pin (14), said guide being formed in particular as an elongated slot (16), said flexion angle appropriately being between 85 and 95 degrees in size.

**Revendications**

1. Endoprothèse pour une articulation de genou, comprenant une partie fémorale (6), une partie tibiale (8) et une partie centrale (18) qui est articulée sur la partie fémorale au moyen d'une broche (14) disposée de préférence horizontalement, la partie fémorale pouvant osciller par rapport à la partie tibiale (8) en tournant autour d'un axe de flexion sensiblement horizontal et présentant un élément de portée courbe (58), l'endoprothèse comprenant aussi un tourillon de centrage (20) agencé entre la partie centrale (18) et la partie tibiale (8) et, autour duquel la partie centrale (18) peut tourner par rapport à la partie tibiale et, en outre, une butée variable en fonction de l'angle de flexion pour arrêter la partie fémorale (6), dont le mouvement de rotation par rapport au tourillon de centrage (20) est ainsi limité dans la position d'extension, *caracterisée en ce qu'* il est prévu, entre la partie fémorale (6) et la partie centrale (18), une partie de portée (54) qui joue le rôle d'un ménisque artificiel, *en ce que* la partie de portée (54) présente un premier corps de guidage (56) qui est en prise avec l'élément de portée circulaire (58) de la partie fémorale (6), *en ce que* la partie de portée (54) est disposée mobile en translation par rapport à la partie centrale (18), dans un plan notamment orthogonal au tourillon de centrage (20), et présente un deuxième corps de guidage (60) qui est en prise avec un chemin de guidage (62) de la partie centrale (18), *en ce que* la broche (14) est disposée excentriquement par rapport à l'axe central (23) de l'élément de portée (58) de la partie fémorale, l'axe de la broche étant déporté vers l'arrière relativement à l'axe central, *et en ce que,* lors de la flexion, la broche peut se déplacer dans un évidement (16) de la partie centrale (18) le long d'une courbe en développante.

2. Endoprothèse selon la revendication 1, *caractérisée en ce que* l'élément de portée (58) de la partie fémorale (6) et les corps de guidage (56) de la partie de portée (54) sont disposés coaxialement à l'axe central (23), la broche (14) étant disposée excentriquement à l'axe central (23) à une distance (25) de cet axe.

3. Endoprothèse selon la revendication 1 ou 2, *caractérisée en ce que,* pour former une butée variable en fonction de l'angle de flexion, la partie de portée (54) présente au moins un ergot (64) qui est engagé dans un chemin de guidage (106) de la partie tibiale (8) ou inversement.

4. Endoprothèse selon une des revendications 1 à 3, *caractérisée en ce que* le chemin de guidage (62) de la partie centrale (18) est disposé sensiblement parallèlement à l'axe de flexion *et/ou en ce que* la partie fémorale (6) présente une cage intracondylaire (22) ainsi que deux rouleaux de condyles (24) disposés latéralement, et qui portent chacun un élément de portée (58).

5. Endoprothèse selon une des revendications 1 à 4, *caractérisée en ce que* la cage intracondylaire (22) présente une section sensiblement en U, avec une ouverture (26) qui s'ouvre vers la partie tibiale (8) *et/ou en ce que* la cage (22) est vissée et fixée au moyen de plaques de compression (68) disposées dans des fentes du condyle.

6. Endoprothèse selon une des revendications 1 à 5, *caractérisée en ce que* les corps de guidage (56, 60) comprennent des roulements *et/ou en ce que* le tourillon de centrage (20) est monté rotatif dans la partie tibiale (8) au moyen de roulements qui sont de préférence constitués par des roulemets à billes axiaux et radiaux (50, 52).

7. Endoprothèse selon une des revendications 1 à 6, *caractérisée en ce que* la première partie de l'articulation, ou partie fémorale (6), et/ou la deuxième partie de l'articulation ou partie tibiale (8) présentent une cage (22, 32), *en ce que* la cage (22, 32) présente un perçage destiné à recevoir un tirant (10, 12), *en ce que* le tirant (10, 12) est en appui par une surface de portée sphérique contre une surface de configuration correspondante de la cage (22, 32), *et en ce que* l'extrémité libre du tirant (10, 12) est vissée dans un corps de vis (38) qui présente sur sa surface extérieure un filetage auto-taraudant pour l'ancrage dans l'os.

8. Endoprothèse selon une des revendications 1 à 7, *caractérisée en ce que* la cage (32) prend appui dans l'os au moyen de griffes d'ancrage (44, 96) et/ou présente des griffes (40) du moins sur une partie de sa surface extérieure, *en ce que* ces griffes d'ancrage (44, 96) sont guidées dans des guidages (66, 98) de la cage (32), qui sont en particulier constituées par des guidages à profil de queue d'aronde, *et en ce que* la distance séparant les griffes d'ancrage (44, 96) de la cage (32) peut être fixée au moyen de vis de serrage (36, 100).

9. Endoprothèse selon une des revendications 1 à 8, *caractérisée en ce que* la partie centrale (18) présente, sur la face inférieure d'un plateau (92), le tourillon de centrage (20) qui est monté dans le perçage (34) de la cage tibiale (32) au moyen de paliers (50, 52) *et/ou en ce que* la partie centrale (18) présente un bras (28) qui est engagé dans une ouverture (26) de la cage intracondylaire qui, à son extémité supérieure, présente la came de roulement qui est en particulier constituée par le trou allongé (16).

10. Endoprothèse selon une des revendications 1 à 9, *caractérisée en ce que* les deux rouleaux condylaires (24) présentent chacun un perçage (78) prévu excentriquement par rapport à l'axe central (23) du rouleau condylaire (24), destiné à recevoir la broche (14), *et/ou en ce que,* dans l'ouverture (26) de la cage intracondylaire (24), est prévu un roulement (20) monté sur la broche (14) pour assurer son guidage dans le trou allongé (16).

11. Endoprothèse selon une des revendications 1 à 10, *caractérisée en ce que* le mouvement de roulement-glissement qui se produit lors de la flexion est limité, au moyen de guidage de la broche (14), constitué en particulier par un trou allongé (16), à un angle de flexion pouvant être prédéterminé, qui est avantageusement compris entre 85 et 95°.

à 10, *caractérisée en ce que* le mouvement de roulement-glissement qui se produit lors de la flexion est limité, au moyen de guidage de

FIG.1

FIG.2

FIG.3

FIG.4

FIG.5

FIG.6

FIG.7

FIG.8